# DEMANDE DE BREVET EUROPEEN

(11) **EP 1 378 853 A1**
(43) Date de publication de la demande: **07.01.2004**
(21) Numéro de dépôt: 02077665.4
(22) Date de dépôt: 04.07.2002
(51) Int. Cl.: G06F 19/00, A61B 5/00

(54) **Système numérique d'assistance médicale**

(71) Demandeur: GE Medical Systems Global Technology Company LLC, Waukesha, Wisconsin 53188 (US)
(72) Inventeur: Muller, Serge, 78280 Guyancourt (FR); Crepin, Guillaume, 75006 Paris (FR); Soubelet, Elisabeth, 2011 JS Haarlem (NL)
(74) Mandataire: Cabinet Hirsch

(57) **Abrégé**

Un système numérique d'assistance médicale à la prise de décision comprend une interface de réception recevant des données cliniques et des données d'enregistrement physiques ou physiologiques. Le système comprend aussi une mémoire de stockage de règles médicales et une unité d'aide à la détection recevant les données d'enregistrement, l'unité fournissant des données mettant en évidence une pathologie. Un moteur d'inférences dans le système reçoit les données cliniques de l'interface, les règles médicales de la mémoire de stockage des règles médicales, et les données fournies par l'unité d'aide à la détection. Le moteur fournit une recommandation à la prise de décision.

Le système permet de prendre en compte toutes les données sur un patient pour fournir une recommandation au praticien lors d'une étude de ce dernier du patient.

## Description

L'invention concerne un système numérique d'assistance médicale.

Les connaissances en radiologie, et en médecine en général, sont difficiles à acquérir et sont souvent partielles et floues dans les domaines les plus difficiles. Il a été observé que des conclusions différentes ont pu être formulées par des praticiens au sujet de patients présentant des symptômes et des antécédents similaires. Ces différences proviennent de l'importante quantité de données sur les patients que les praticiens ont à considérer ; ces différences proviennent aussi des incertitudes liées aux critères de décision des praticiens. Pour une spécialité médicale donnée, seuls quelques experts sont capables, à partir de recherches poussées, de déterminer et d'utiliser avec de fortes chances de succès des modèles de décision. Ces modèles de décision indiquent quelle procédure appliquer au patient à partir d'une série de données collectée et analysée. Ces données sont par exemple des réponses à des questionnaires (par ex. un historique familial ou personnel, des habitudes ou des symptômes du patient). Les données peuvent aussi être des observations comportant des paramètres descriptifs provenant d'analyses d'imageries médicales du patient, les images étant acquises selon différents procédés (par ex. la forme de microcalcifications dans des mammographies par rayons X, les sur-densités apparaissant sur des images issues de résonance magnétique avec utilisation de produits de contraste , la forme et la texture de signaux échographiques en ultrasons). Les données peuvent aussi provenir d'un examen clinique effectué sur le patient, d'enregistrements métaboliques (ex : électrocardiogramme, encéphalogramme) ou d'analyse cytologique ou histologique de cellules ou de tissus prélevés sur le patient.

Au cours de l'examen de patients, la plupart des praticiens sont en mesure d'obtenir des performances élevées dans leur processus décisionnel, lorsque le cas du patient est typique à diagnostiquer ou lorsque la procédure est unique et largement admise. En revanche, lorsque le cas du patient est atypique ou lorsque le médecin est inexpérimenté, il est parfois difficile de prendre une décision à une étape donnée dans le processus d'examen du patient. Il est souvent fait appel à des experts dans des cas délicats, pour confirmer ou suggérer une décision et l'étape suivante à suivre dans le processus d'examen du patient. Le nombre limité d'experts réduit les possibilités du recours à leur service. De plus, l'avis des experts eux-mêmes est susceptible de varier.

Le document EP-A-1035507 décrit un système d'assistance à la détection assistée par ordinateur (en anglais Computer-Aided Détection ou CAD). Ce système permet de lire une image médicale et de l'analyser pour en extraire des zones suspicieuses traduisant la présence d'une lésion. Seules des informations quantitatives sur les lésions sont fournies.

Il existe donc un besoin d'assister les praticiens à prendre des décisions lors de l'étude d'un patient alors qu'ils ont à considérer une multitude des données concernant le patient.

### BREF RESUME DE L'INVENTION

Selon un mode de réalisation l'invention, un système numérique d'assistance médicale à la prise de décision comprend une interface de réception recevant des données cliniques et des données d'enregistrement physiques ou physiologiques. Le système comprend aussi une mémoire de stockage de règles médicales et une unité d'aide à la détection recevant les données d'enregistrement, l'unité fournissant des données mettant en évidence une pathologie. Un moteur d'inférences dans le système reçoit les données cliniques de l'interface, les règles médicales de la mémoire de stockage des règles médicales, et les données fournies par l'unité d'aide à la détection. Le moteur fournit une recommandation à la prise de décision.

Le système présente les avantages suivants. Le système fournit une recommandation au praticien sur la décision qu'il peut prendre pour poursuivre le processus d'étude du patient. Le système prend en compte toutes les données concernant un patient et les règles utilisées par les praticiens pour prendre une décision. La recommandation fournie par le système évite, par exemple, que le praticien ait recours à une opération du patient, à un stade trop précoce dans le processus d'étude du patient, et qu'il préfère prélever dans un premier temps les échantillons de tissu d'organes par un moyen moins invasif.

### BREVE DESCRIPTION DES DESSINS

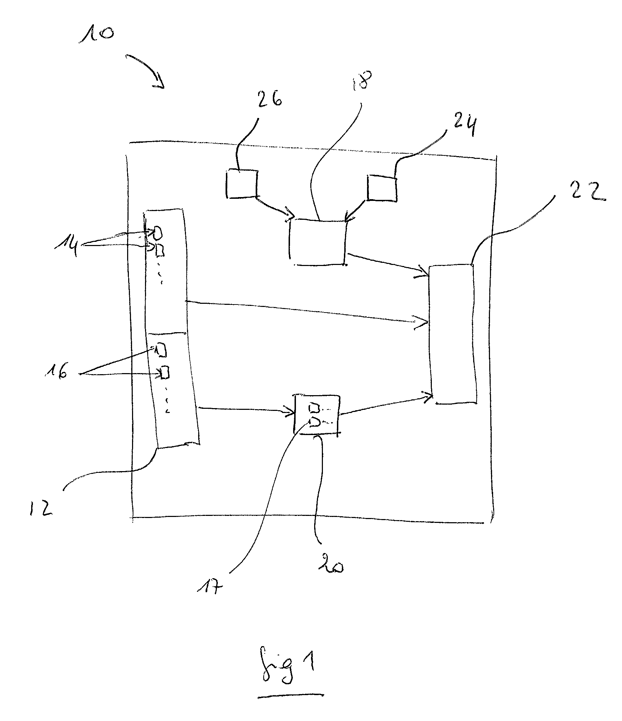

La figure 1 montre une vue schématique d'un système d'assistance médicale.

### DESCRIPTION DETAILLEE DE L'INVENTION

En référence à la figure 1, est décrit un système 10 numérique d'assistance médicale à la prise de décision. La figure 1 montre une vue schématique du système numérique. Le système 10 présente une mémoire 18 de stockage de règles médicales et une interface 12 de réception recevant des données cliniques 14 et des données d'enregistrement 16 physiques ou physiologiques concernant le patient. Le système 10 comprend aussi une unité 20 d'aide à la détection ; l'unité 20 reçoit les données d'enregistrement 16 et fournit en sortie des données 17 mettant en évidence une pathologie. Un moteur d'inférences 22 dans le système 10 reçoit les données cliniques 14, les règles médicales de la mémoire 18 ainsi que les données 17 fournies par l'unité 20 d'aide à la détection. Le moteur 22 fournit une recommandation à la prise de décision.

L'interface 12 de réception reçoit les données cliniques 14 sur le patient qui sont des informations objectives sur le patient. Elles comprennent des informations sur la taille, le poids ou l'âge du patient. Les données cliniques 14 comprennent aussi des informations sur les antécédents cliniques du patient, telles que des maladies antérieurement contractées par le patient. Les données 14 sont par exemple collectées en utilisant une interface de réseau avec un questionnaire qui peut être rempli par le patient ; les données 14 peuvent aussi être collectées par le praticien qui se connecte à un réseau de telle sorte qu'il lui est possible de recevoir des données d'autres ordinateurs.

L'interface 12 reçoit par ailleurs les données d'enregistrement 16 physiques ou physiologique du patient. Les données 16 sont également des informations sur le patient mais qui sont sujettes à des interprétations variables selon les praticiens. Les données 16 peuvent être par exemple des observations faites par le praticien sur le patient durant des examens physiques. Les données peuvent aussi être des données issues d'un appareil d'imagerie médicale. L'appareil d'imagerie délivre par exemple des images avec un format 2D, 3D (volume, image et temps) ou 4D (par ex. volume et temps). Des images peuvent être fournies par plusieurs appareils d'imagerie, qui délivrent des images en utilisant différentes propriétés de l'interaction entre radiation et lésions. Les images sont par exemple des images issues de scanner ou d'images radiologiques ou échographiques qui fournissent une description morphologique du patient ; les images peuvent également provenir d'appareils de médecine nucléaire, d'IRM fonctionnelle ou de tomographie à émission de positrons.

Les données sont stockées dans une banque de données, par exemple dans une mémoire vive RAM. A chaque étape de l'étude du patient, le praticien peut entrer de nouvelles données 14, 16.

La mémoire 18 de stockage stocke les règles médicales du praticien. Les règles médicales sont utilisées par les praticiens ou les experts médicaux dans leurs études de familles de maladies. Les règles médicales sont un modèle de décision que le praticien applique pour prendre ses décisions. La mémoire 18 de stockage de règles peut comprendre le modèle de décision du praticien utilisateur, établi sur la base de sa propre expertise accumulée par son expérience. De manière avantageuse, les règles peuvent aussi être des règles établies par un expert du domaine médical concerné. Le système 10 présente l'avantage de permettre à un praticien inexpérimenté d'utiliser la recommandation du système 10 comme un guide et de profiter des connaissances d'experts.

Des règles instituées par une pluralité d'experts médicaux peuvent aussi être intégrées dans la mémoire 18. Le praticien peut alors obtenir une recommandation en sélectionnant un type de règles médicales. Le praticien peut aussi obtenir une pluralité de recommandations qu'il peut comparer entre elles.

L'unité 20 d'aide à la détection (en anglais Computer-Aided Détection ou CAD) reçoit en entrée les données 16 ; en sortie, l'unité 20 fournit les données 17 mettant en évidence l'existence d'une pathologie. Les données 16 sont des informations concernant le patient et sur lesquelles le praticien s'appuie pour prendre une décision pour passer à une prochaine étape du processus d'examen du patient. Les données 17 fournies en sortie sont des mesures quantitatives sur l'existence de pathologies. Par exemple, lorsque les données 16 fournies en entrée sont issues de données fournies par un appareil d'imagerie médicale, l'unité 20 lit les images, les analyse et en extrait des zones suspicieuses traduisant la présence d'une lésion. Les données 17 peuvent comprendre un index de malignité traduisant le risque que la lésion soit cancéreuse. Les données 17 peuvent aussi comprendre une localisation de lésions sur l'organe d'un patient. L'unité 20 permet ainsi de synthétiser les données fournies par exemple par l'imagerie médicale et d'assister le praticien pour améliorer la qualité de la détection ou de la caractérisation lors de l'analyse des images. L'unité 20 permet au système 10 de prendre les données cliniques 14 en considération ensemble avec les données 16 d'enregistrement.

Le moteur d'inférence 22 fournit une recommandation au praticien pour l'assister dans sa prise de décision. Le moteur d'inférence exploite la base d'informations comprenant les données décrivant le patient. Ces données comprennent les données cliniques 14 reçues de l'interface 12 et les données 17 fournies par l'unité 20. Le moteur 22 reçoit aussi les règles médicales de la mémoire 18 ; à partir des règles médicales, et sur la base des données 14 et 17 le moteur 22 simule les raisonnements et les décisions du praticien. Le moteur d'inférence 22 est par exemple d'ordre zéro : il fournit une recommandation au praticien en effectuant le raisonnement du praticien. Le moteur d'inférence 22 peut aussi être d'ordre 1 ; le moteur 22 reconsidère l'ensemble du processus décisionnel arborescent pour valider la considération d'une hypothèse qui n'était pas la plus probable lors d'une étape antérieure dans l'examen du patient. A partir d'un ensemble de données acquises sur le patient, le praticien peut être amené à considérer plusieurs hypothèses de pathologies avec des degrés de malignité différents qui conduisent à autant de décision thérapeutiques. Le parcours des données jusqu'aux hypothèses cliniques peut être représenté sous forme d'un arbre de décision. Une donnée supplémentaire peut modifier le nombre d'hypothèses à considérer et le poids relatif de ces hypothèses. L'arbre de décision utilisé précédemment est alors modifié. D'autres technologies telles que les arbres flous peuvent être choisies pour l'implémentation des règles. Des réseaux de neurones artificiels peuvent aussi être utilisés pour construire un processus décisionnel arborescent à partir de la connaissance de données collectées pendant l'examen.

La recommandation fournie par le moteur 22 du système 10 est une indication pour le praticien de la prochaine étape qui peut être appliquée sur le patient. La recommandation fournie par le système 10 optimalise la probabilité que la décision du praticien ait un impact positif sur la santé du patient. La recommandation est par exemple l'invitation à effectuer de nouvelles images médicales pour obtenir d'autres données sur le patient, une biopsie à l'aiguille, à recourir à une étape de chirurgie, ou l'administration de drogues spécifiques (chimiothérapie). Le système 10 peut compléter la recommandation par un index de confiance ou de probabilité d'efficacité. L'index est par exemple établi par le moteur d'inférence 22 lors des différentes étapes de la simulation du raisonnement par un processus arborescent. Le système peut aussi fournir une pluralité de recommandations en délivrant pour chacune des recommandations une probabilité d'efficacité. La recommandation fournie par le système réduit les variations entre les décisions prises par différents praticiens dans l'étude du patient.

Le système 10 numérique est par exemple un ordinateur.

Avantageusement, le système 10 comprend en outre une interface 24 d'enrichissement en règles médicales de la mémoire 18 de stockage de règles médicales. Les règles médicales peuvent être stockées sur la mémoire 18 avant d'utiliser le système 10 sur plusieurs patients ; la mémoire 18 peut aussi être remise à jour avec de nouvelles règles médicales à chaque étape de l'étude d'un patient. Les nouvelles règles sont par exemple chargées depuis un réseau ou à partir d'organes d'archivage tels que un CD-ROM. Ceci peut être réalisé, par exemple, en utilisant une interface de réseau qui permet l'introduction d'hypothèses, de règles et de conclusions en langage naturel ou qui les charge à partir de support d'enregistrement (par exemple des disquettes ou des CD-ROM). Un interpréteur assure la traduction d'entrée en langage naturel en règles qui peuvent être exécutées par un ordinateur (par exemple en utilisant un langage tel que Prolog).

Le système peut comprendre en outre un module 26 de déduction de règles médicales à partir de la comparaison entre la vérité sur la pathologie obtenue a posteriori - après analyse anatomo-pathologique par exemple - et les données en entrée de l'interface 12 et en sortie de l'unité 20, le module 26 pouvant fournire les règles médicales à la mémoire 18 de stockage de règles médicales. On peut utiliser par exemple, un mécanisme d'apprentissage supervisé qui, à partir de données sur le patient et de la vérité obtenue a posteriori, permet de mettre à jour les règles de décision de la structure de décision arborescente. En particulier, on peut utiliser un réseau de neurones artificiel de type perceptron pour lequel l'apprentissage permet de mettre à jour le poids de ses connexions. Les nouvelles règles construites complètent la mémoire 18 de stockage de règles médicales.

Le système trouve, par exemple, une application en mammographie.

## Revendications

1. Un système numérique d'assistance médicale à la prise de décision, comprenant :
- une interface (12) de réception recevant :
- des données cliniques (14), et
- des données d'enregistrement (16) physiques ou physiologiques,
- une mémoire (18) de stockage de règles médicales,
- une unité (20) d'aide à la détection recevant les données d'enregistrement (16) et qui fournit des données (17) mettant en évidence une pathologie,
- un moteur d'inférences (22) recevant :
- les données cliniques (14) de l'interface (12),
- les règles médicales de la mémoire (18) de stockage des règles médicales, et
- les données (17) fournies par l'unité (20) d'aide à la détection, le moteur (22) fournissant une recommandation à la prise de décision.

2. Le système de la revendication 1, **caractérisé en ce que** les données cliniques (14) comprennent le sexe, la taille, le poids, l'âge, l'histoire familiale et les antécédents cliniques d'un patient.

3. Le système de la revendication 1 ou 2, **caractérisé en ce que** les données d'enregistrement (16) comprennent des données issues d'un appareil d'imagerie médicale.

4. Le système de la revendication 3, **caractérisé en ce que** les données (17) comprennent un index de malignité.

5. Le système de la revendication 3 ou 4, **caractérisé en ce que** les données (17) comprennent une localisation de lésions sur l'organe d'un patient.

6. Le système de l'une des revendications 3 à 5, **caractérisé en ce que** les données (17) comprennent les caractéristiques de lésions sur l'organe d'un patient.

7. Le système de l'une des revendications 1 à 6, **caractérisé en ce que** le système comprend en outre une interface (24) d'enrichissement en règles médicales de la mémoire (18) de stockage de règles médicales.

8. Le système de l'une des revendications 1 à 7, **caractérisé en ce que** le système comprend en outre un module (26) de déduction de règles médicales à partir de la comparaison entre la vérité sur la pathologie obtenue a posteriori et les données en entrée de l'interface (12) et en sortie de l'unité (20), le module (26) fournissant les règles médicales à la mémoire (18) de stockage de règles médicales.
